# EUROPEAN PATENT APPLICATION

(11) **EP 4 427 794 A1**
(43) Date of publication of application: **11.09.2024**
(21) Application number: 22904473.0
(22) Date of filing: 03.11.2022
(51) Int. Cl.: A61M 37/00

(54) **MICRONEEDLE ASSEMBLY**

(30) Priority: 07.12.2021 KR 20210174179
(71) Applicant: Daewoong Therapeutics Inc., Suwon-si, Gyeonggi-do 16226 (KR)
(72) Inventor: KANG, Yoon Sik, Seoul 06013 (KR); IM, Ji Yeon, Daejeon 34308 (KR); EUM, Jae Hong, Uiwang-si Gyeonggi-do 16021 (KR); LEE, Boo Yong, Gimpo-si Gyeonggi-do 10078 (KR); KIM, Dong Hwan, Suwon-si Gyeonggi-do 16336 (KR); KANG, Bok Ki, Seongnam-si Gyeonggi-do 13539 (KR)
(74) Representative: RGTH
(86) International application number: PCT/KR2022/017102
(87) International publication number: WO 2023/106639

(57) **Abstract**

An example embodiment of the present disclosure provides a microneedle assembly including: a mold having one or more concave portions formed in an upper surface thereof and microneedles molded by being injected into the one or more concave portions; and an applicator detachably coupled to the upper surface of the mold, in which the applicator is formed in a plate shape, and has a first surface which protects the microneedles so that the microneedles are not to be exposed during packaging and a second surface on which an adhesion portion is formed to be attached to the microneedles during use.

## Description

### TECHNICAL FIELD

The present disclosure relates to a microneedle assembly, and more specifically, to a microneedle assembly which is capable of safely keeping microneedles and applying the microneedles to a skin in a convenient manner while preventing the microneedles from being contaminated during use.

### BACKGROUND

Delivery of pharmaceutical agents to a skin using dissolvable microneedles is greatly affected by mechanochemical properties such as a shape, physical property and solubility of the dissolvable microneedles, as well as application conditions such as a dosage (insertion) state and a dosage time. Accordingly, under inappropriate application conditions, the dissolvable microneedles may not be sufficiently inserted into a dermal layer of the skin, and therefore, a fixed quantity of pharmaceutical agents may not be delivered to the skin.

As an example, in a case where an unskilled user presses the microneedles at a certain pressure to insert the same into the skin, when such a pressure is not enough for the microneedles to penetrate the skin, some of the microneedles may not be sufficiently inserted into the dermal layer of the skin. In addition, when the pressure is applied unevenly over the entire area of an array of the microneedles, or when a force is applied in a shear direction, the microneedles may be inserted unevenly into the skin. Further, even if all the microneedles are sufficiently inserted into the skin at strong pressure, when the pressure is not continuously applied, an aspect ratio of each microneedle may be decreased due to dissolution of a polymer, and the array of the microneedles may be displaced due to an elasticity of the skin itself and an external movement.

In addition, when the user inserts the microneedles into the skin by taking out a microneedle patch from a storage container, attaching the same to the skin, and pressing the microneedle patch while rubbing the microneedle patch, the microneedles may not be inserted perpendicularly into the skin or may be bent. Further, the microneedles may be contaminated by user's fingers.

In order to solve the above matters, there is a need for a technique for safely keeping microneedles, preventing the microneedles from being contaminated during use, and minimizing a variation in dosage of pharmaceutical agents by users.

### [Document in Related Art]

Korean Patent Application Registration No. 10-1033514 (April 29, 2011)

The present disclosure was made for the purpose of solving the above matters, and the present disclosure is for the purpose of a microneedle assembly which is capable of safely keeping microneedles and applying the microneedles to a skin in a convenient manner while preventing the microneedles from being contaminated during use.

An example embodiment of the present disclosure provides a microneedle assembly which may include: a mold having one or more concave portions formed in an upper surface thereof and microneedles molded by being injected into the one or more concave portions; and
an applicator detachably coupled to the upper surface of the mold,
wherein the applicator is formed in a plate shape, and has a first surface which protects the microneedles so as not to be exposed during packaging and a second surface on which a adhesion portion is formed to be attached to the microneedles during use so that the microneedles are separated from the mold.

In an aspect, the applicator may further include one or more pressing protrusions provided at positions corresponding to the microneedles on the second surface, and having one end portions formed to be flat, and the adhesion portions may be formed on the one end portions of the one or more pressing protrusions.

In an aspect, the mold may further include one or more guide protrusions formed outside an area where the microneedles are formed. The applicator may be formed outside an area where the one or more pressing protrusions are formed, and may further include one or more guide holes into which the one or more guide protrusions are inserted. The mold and the applicator may be coupled to each other by the one or more guide protrusions and the one or more guide holes during packaging. The one or more guide protrusions and the one or more guide holes may be configured to guide the adhesion portion toward the microneedles during use.

In an aspect, the microneedle assembly may further include a protection cover configured to be detachably coupled to the second surface of the applicator during packaging and temporarily close the second surface.

In an aspect, the applicator may be made of a sturdy material.

In an aspect, the one or more concave portions of the mold may be sharp-tipped concave portions.

In an aspect, the microneedles may be provided independently of each other.

In an aspect, the mold may be a primary packaging container in a full close contact with the microneedles and may be configured to be removed before the microneedles are applied to a human body.

In an aspect, the one or more guide holes may be formed to penetrate from the first surface to the second surface of the applicator.

According to a microneedle assembly of one aspect of the present disclosure, microneedles may be kept in a safe manner so as not to be exposed to the outside, and may be applied to a skin in a convenient manner using an applicator during use.

Further, the applicator includes one or more pressing protrusions and one or more adhesion portions provided at positions corresponding to the microneedles formed in a mold. This makes it possible to more easily separate the microneedles from the mold and penetrate the microneedles to the skin with uniform pressure.

Further, by adjusting heights of the pressing protrusions of the applicator, depths at which the microneedles are inserted into the skin may be ensured.

Effects of the present disclosure are not limited to the aforementioned effects, and other effects may be inferred from the configurations described in the detailed description or the claims of the present disclosure.

### BRIEF DESCRIPTION OF THE DRAWINGS

FIG. 1A is a perspective view of a microneedle assembly according to an example embodiment of the present disclosure, and FIG. 1B is a cross-sectional view taken along line A-A' in FIG. 1A.
FIG. 2A is an exploded perspective view of the microneedle assembly according to the example embodiment of the present disclosure, and FIG. 2B is a cross-sectional view taken along line B-B' in FIG. 2A.
FIG. 3A is a perspective view of a mold and an applicator before microneedles are separated from the mold in an example embodiment of the present disclosure, and FIG. 3B is a cross-sectional view taken along line C-C' in FIG. 3A.
FIG. 4A is a perspective view of the mold and the applicator when the microneedles are attached to the mold in an example embodiment of the present disclosure, and FIG. 4B is a cross-sectional view taken along line D-D' in FIG. 4A.
FIG. 5A is a perspective view of the mold and the applicator after the microneedles are separated from the mold in an example embodiment of the present disclosure, and FIG. 5B is a cross-sectional view taken along line E-E' in FIG. 5A.
FIGS. 6A and 6B are state diagrams illustrating states before and after the microneedles is applied to the human body using the applicator according to an example embodiment of the present disclosure, respectively.

### DETAILED DESCRIPTION

The present disclosure will be described below with reference to the accompanying drawings. The present disclosure may be embodied in many different forms and is not limited to the example embodiments described herein. In order to clearly describe the present disclosure, detailed descriptions of parts irrelevant to the present disclosure will be omitted, and the same reference numerals will be given to the same constituent elements throughout the specification.

Throughout the specification, when one constituent element is referred to as being "connected" to another constituent element, the one constituent element may be "directly connected" to another constituent element, or may be "indirectly connected" to another constituent element by intervening yet another constituent element therebetween. Further, when one constituent element "comprise or includes" another constituent element through the specification, this means that the one constituent element may further include other constituent elements, rather than excluding other constituent elements, unless other stated.

Example embodiments of the present disclosure will be described in detail below with reference to the accompanying drawings.

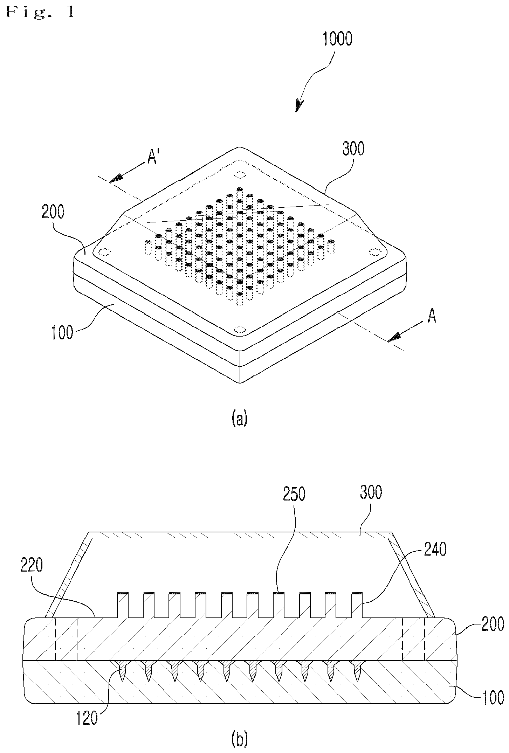

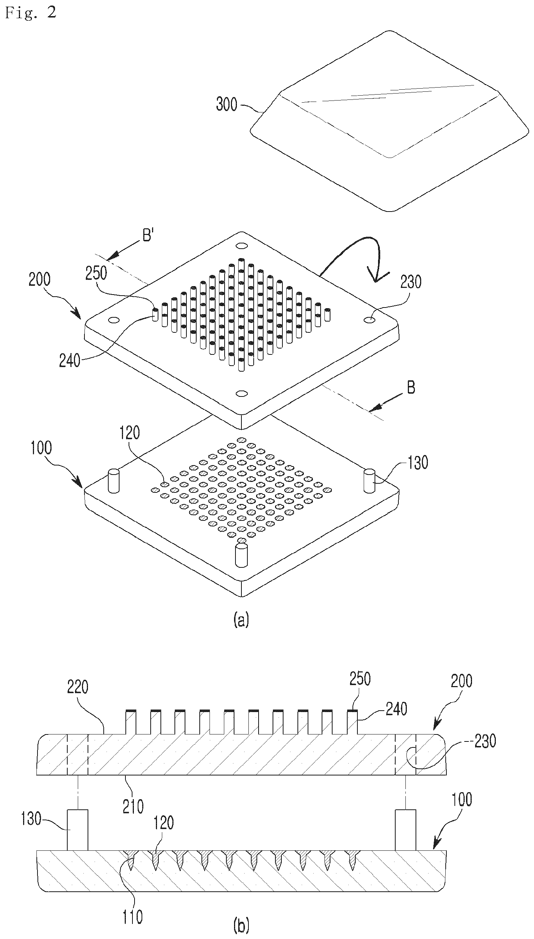

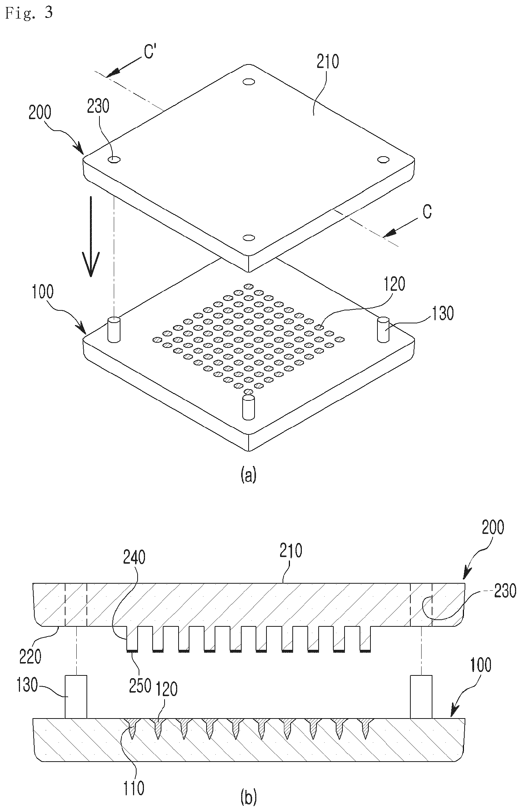

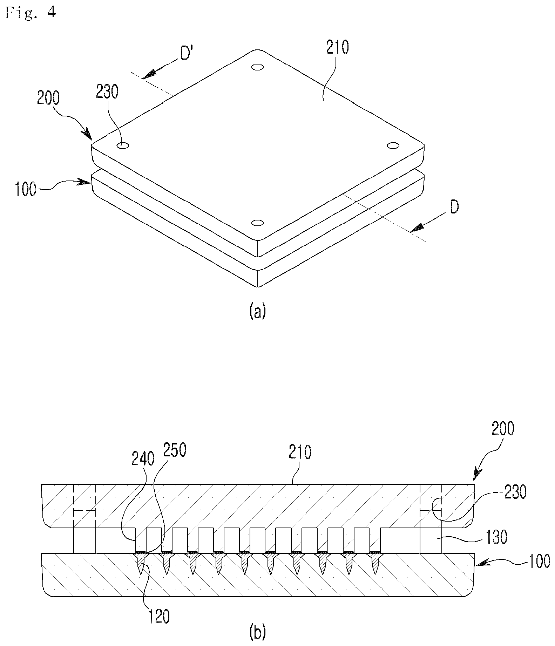

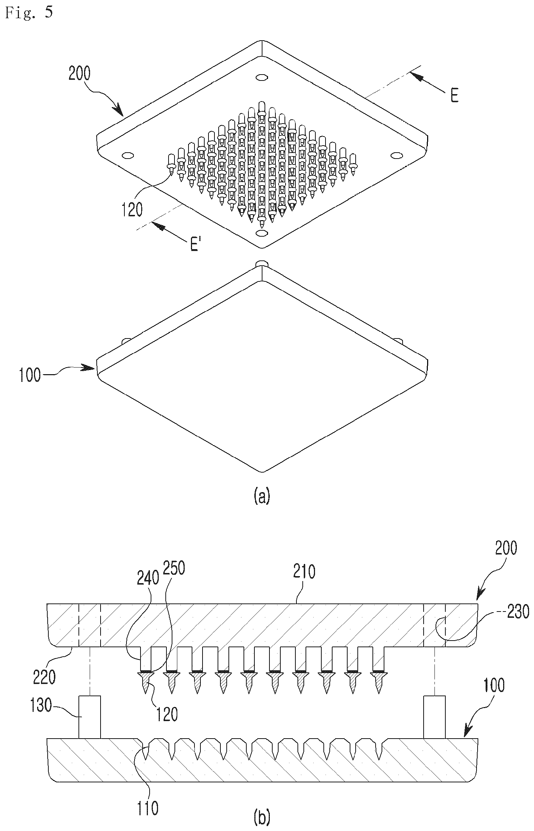

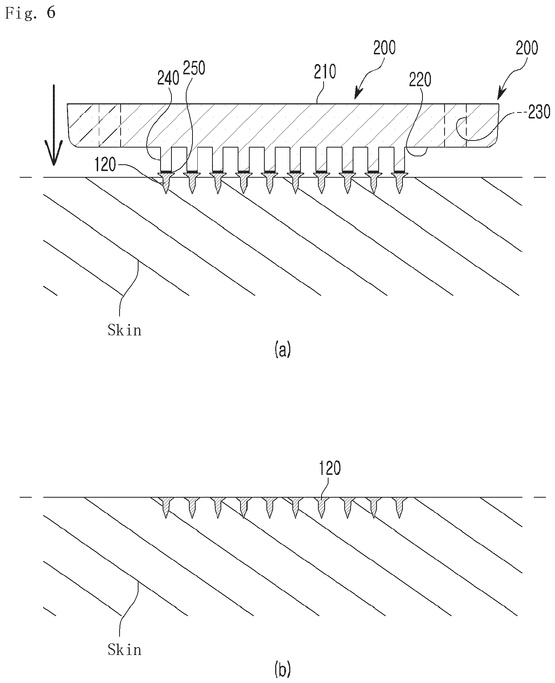

FIG. 1A is a perspective view of a microneedle assembly according to an example embodiment of the present disclosure, and FIG. 1B is a cross-sectional view taken along line A-A' in FIG. 1A. FIG. 2A is an exploded perspective view of the microneedle assembly according to the example embodiment of the present disclosure, and FIG. 2B is a cross-sectional view taken along line B-B' in FIG. 2A. FIG. 3A is a perspective view of a mold and an applicator before microneedles are separated from the mold in an example embodiment of the present disclosure, and FIG. 3B is a cross-sectional view taken along line C-C' in FIG. 3A. FIG. 4A is a perspective view of the mold and the applicator when the microneedles are attached to the mold in an example embodiment of the present disclosure, and FIG. 4B is a cross-sectional view taken along line D-D' in FIG. 4A. FIG. 5A is a perspective view of the mold and the applicator after the microneedles are separated from the mold in an example embodiment of the present disclosure, and FIG. 5B is a cross-sectional view taken along line E-E' in FIG. 5A. FIGS. 6A and 6B are state diagrams illustrating states before and after the microneedles is applied to the human body using the applicator according to an example embodiment of the present disclosure, respectively.

Referring to FIGS. 1A to 6B, a microneedle assembly 1000 of the present disclosure includes a mold 100, an applicator 200, and a protection cover 300.

More specifically, the microneedle assembly 1000 is provided with the mold 100 including one or more concave portions 110 formed in an upper surface thereof and microneedles 120 injected into the concave portions 110 to be molded therein, and the applicator 200 detachably coupled to the upper surface of the mold 100. In an example embodiment, the applicator 200 is formed in a plate shape. The applicator 200 has a first surface 210 for protecting the microneedles 120 from being exposed during packaging, and a second surface 220 to be attached to the microneedles 120 when the applicator 200 is used. The adhesion portion 250 is formed on the second surface 220 to separate the microneedles 120 from the mold 100.

The mold 100 may have one or more concave portions 110 formed in the upper surface thereof. A lower surface of the mold 100 may be flat. Each of the concave portions 110 acts as a mold for each microneedle 120 and may have a shape corresponding to the microneedle 120.

In this case, each concave portion 110 may be a fine-tipped concave portion. As used herein, the term "fine-tipped concave portion" means a concave portion depressed downward from a flat upper surface such that a cross-sectional area thereof is reduced from the flat upper surface toward a lower surface forming a tip, like a shape of the typical microneedle 120. The term "upper surface" and/or "lower surface" used herein is for specifying a relative positional relationship in each configuration and does not specify absolute positions thereof.

The concave portions 110 may be provided in the form of an array independently of each other on the upper surface of the mold 100 without being connected to each other in the upper surface of the mold 100. With this configuration, the microneedles 120 molded by being injected into the concave portions 110 may also be provided in the form of an array independently of each other without being connected to each other. The microneedles 120 may be separated from the respective concave portions 110 of the mold 100 by the adhesion portion 250 of the applicator 200.

The microneedles 120 may be formed by molding a raw material containing one or more selected from the group consisting of a non-metal, a biodegradable polymer, and pharmaceutical ingredients with the concave portions 110 of the mold 100.

According to an example embodiment, the mold 100 may be made of a solid material. The mold 100 may be made of any suitable material, preferably a plastic resin.

More specifically, the mold 100 may be made of at least one or more selected from the group consisting of polyethyleneterephthalate (PET), polyvinylchloride (PVC), and polypropylene (PP), but is not limited thereto.

A microneedle made of a silicon material in the related art is expensive, thus the silicon microneedle in the related art often needs to be reused. In the reusing process of the silicon microneedle, verification of a cleaning validation (CV) of fine concave portions in which microneedles are molded may be a difficult process to conduct. Verification of a permeability of a cleaning solvent to silicon may also be a difficult process. Further, the silicon material is physicochemically decomposable and has a relatively weak hardness. As a result, fragments may be generated during a manufacturing or cleaning process. In addition, it is difficult to determine a lifespan of a mold made of the silicon material.

In addition, in the case in which the mold is manufactured using the silicon material, the silicon does not play a protective role for ambient air or external foreign matters. As a result, dried microneedles need to be removed from the mold to repackage the same. In this case, since it is practically difficult to individually pack each of the fine-sized microneedles in an airtight manner, the microneedles may be packaged in a pouch or a container while being exposed to ambient air.

The sealability and stability of a packaging container have a significant effect on maintaining the quality of pharmaceuticals in the packaging container. Therefore, the packaging container needs to completely prevent physicochemical deformation or deterioration of the pharmaceuticals in the packaging container through a transport condition simulation and mechanical test relating to final products.

The mold 100 of the present disclosure may function as a primary packaging container in a full close contact with the microneedles 120 without removing and repackaging the microneedles 120 molded in the concave portions 110. The mold 100 may be configured to be removed before use. This makes it possible to simplify an operation of packaging the microneedles and easily maintain the quality of the microneedles.

The applicator 200 is configured to separate the microneedles 120 from the mold 100 and allow the separated microneedles 120 to penetrate into the skin of the human body. The applicator 200 may be formed in a plate shape and may have the first surface 210 and the second surface 220.

The first surface 210 covers the upper surface of the mold 100 during packaging to protect the microneedles 120 from being exposed to the outside.

The second surface 220 has one or more adhesion portions 250 formed at positions corresponding to the microneedles 120. When the applicator 200 is used, the adhesion portions 250 adhere to the microneedles 120 so as to separate the microneedles 120 from the mold 100.

The applicator 200 may be made of a solid material. The applicator 200 may be made of any suitable material, preferably a plastic resin.

More specifically, the applicator 200 may be made of at least one or more selected from the group consisting of polyethyleneterephthalate (PET), polyvinylchloride (PVC), and polypropylene (PP), but is not limited thereto.

According to an example embodiment, the applicator 200 may further have one or more pressing protrusions 240 provided at positions corresponding to the respective microneedles 120 on the second surface 220. One end portion of each pressing protrusion 240 is flat.

The pressing protrusion 240 may be formed in a cylindrical shape, but is not limited thereto. As an example, the pressing protrusion 240 may be formed in a polygonal pillar shape such as a triangular pillar or square pillar.

The pressing protrusions 240 are formed to be the same as the number of the microneedles 120 at the positions corresponding to the microneedles 120 on the second surface 220. Each adhesion portion 250 may be formed on one end portion of the pressing protrusion 240.

That is, the applicator 200 is configured such that the pressing protrusions 240 are provided at the positions corresponding to the microneedles 120. This makes it possible to easily separate the microneedles 120 from the mold 100 and let the array of the microneedles 120 penetrate into the skin with uniform pressure throughout.

Further, the applicator 200 may set a depth at which the microneedle 120 is inserted into the skin with a height of the pressing protrusion 240.

The applicator 200 may be detachably coupled to the upper surface of the mold 100.

According to an example embodiment, the mold 100 may further include one or more guide protrusions 130 formed outside an area where the microneedles 120 are formed on the upper surface of the mold 100. Further, the applicator 200 may further include one or more guide holes 230 formed outside an area where the pressing protrusions 240 are formed. The guide protrusions 130 are inserted into the respective guide holes 230. In this configuration, by the guide protrusions 130 and the guide holes 230, the mold 100 and the applicator 200 are coupled to each other during packaging. When the applicator 200 is used, the adhesion portion 250 may be guided toward the respective microneedles 120.

In other words, when the first surface 210 of the applicator 200 is arranged to cover the upper surface of the mold 100 during packaging, the guide protrusions 130 are inserted into the respective guide holes 230 so that the mold 100 and the applicator 200 are coupled to each other. In addition, when the applicator 200 is used, the guide protrusions 130 are guided into the respective guide holes 230 so that the adhesion portion 250 formed on the pressing protrusions 240 of the applicator 200 easily adhere to the respective microneedles 120.

According to an example embodiment, the guide protrusion 130 may be formed in a circular pillar shape, but is not limited thereto. As an example, the guide protrusion 130 may be formed in a polygonal pillar shape such as a triangular pillar or square pillar. Further, the guide hole 230 may be formed in a shape corresponding to the guide protrusion 130.

In an example embodiment, the guide hole 230 may be formed to penetrate from the first surface 210 to the second surface 220 of the applicator 200. With this configuration, the guide protrusion 130 may be inserted into the guide hole 230 on both the first surface 210 and the second surface 220 of the applicator 200, that is, in both directions of the applicator 200.

In a case where a binding force between a lateral side of the microneedle 120 and the concave portion 110 is greater than an adhesive force between the adhesion portion 250 and an upper surface of the microneedle 120, when the microneedle 120 is separated from the concave portion 110 for application to the skin, the microneedle 120 may not be fully separated from the concave portion 110. This makes it difficult to dose a required amount of active pharmaceutical ingredients contained in the microneedle 120 into the skin.

Therefore, in order to make the adhesive force between the adhesion portion 250 and the upper surface of the microneedle 120 greater than the binding force between the lateral side of the microneedle 120 and the concave portion 110, a composition of the adhesion portion 250 and the resulting adhesive force may be adjusted, and/or the concave portion 110 in contact with the microneedle 120 may be coated with a certain material and/or a surface thereof may be treated to have a certain level of roughness. With this configuration, it is possible to decrease the binding force between the lateral side of the microneedle 120 and the concave portion 110, thus easily separating the microneedle 120 from the concave portion 110.

According to an example embodiment, the microneedle assembly 1000 may further include a protection cover 300 detachably coupled to the second surface 220 of the applicator 200 during packaging and configured to temporarily close the second surface 220.

The protection cover 300 may have a structure in which a lower portion is open. The protection cover 300 may be formed to have an internal depth more inwardly depressed than the heights of the pressing protrusions 240 so as not to come contact with the pressing protrusions 240 of the applicator 200 during packaging. Further, the protection cover 300 may be bonded or coupled to the applicator 200 along an edge of the protection cover 300.

Meanwhile, referring to FIGS. 1 to 6, a method of using the microneedle assembly 1000 is as follows.

First, in order to apply the microneedle 120 to a specific skin of a user, the protection cover 300 temporarily adhered or coupled to cover the applicator 200 is removed to expose the applicator 200 provided on the mold 100 to the outside.

Subsequently, the applicator 200 coupled to the mold 100 to cover the upper surface of the mold 100 is separated from the mold 100. That is, force is applied to move the applicator 200 away from the mold 100 so that the guide protrusions 130 are separated from the respective guide holes 230.

Subsequently, the applicator 200 is turned over and arranged such that the pressing protrusions 240 and the adhesion portions 250 formed on the respective pressing protrusions 240 face the mold 100 in which the microneedles 120 are formed.

Thereafter, the applicator 200 is moved toward the mold 100 such that the adhesion portions 250 are attached to the microneedles 120, respectively. In this case, the movement of the applicator 200 to the mold 100 may be guided by the guide holes 230 and the guide protrusions 130.

Subsequently, the microneedles 120 attached to the adhesion portions 250 are separated from the concave portions 110 of the mold 100. Thereafter, the first surface 210 of the applicator 200 is pressed for the microneedles 120 to penetrate into the skin. As an example, the applicator 200 may be made of a solid material. By the pressing protrusions 240 provided in the applicator 200, the microneedles 120 may penetrate into the skin with uniform pressure over the entire area.

The foregoing description of the present disclosure is merely an example. It will be understood by those skilled in the art that variations are readily possible in other specific forms without changing the technical ideas or essential features of the present disclosure. Therefore, it should be noted that the example embodiments described above are exemplary in all respects and are not restrictive. For example, each constituent element that is described in a single form may be embodied in a distributed fashion. Similarly, constituent elements that are described in the distributed fashion may be embodied in a combined fashion.

The scope of the present disclosure should be construed within the appended claims rather than the foregoing detailed description, and it is intended that all changes or modifications that come from the meaning and scope of the claims and their equivalent concept be embraced therein.

### EXPLANATION OF REFERENCE NUMERALS

] 1000: Microneedle assembly
100: Mold
110: Concave portion
120: Microneedle
130: Guide protrusion
200: Applicator
210: First surface
220: Second surface
230: Guide hall
240: Pressing protrusion
250: Adhesion portion
300: Protection cover

## Claims

1. A microneedle assembly, comprising:
a mold having one or more concave portions formed in an upper surface thereof and microneedles molded by being injected into the one or more concave portions; and
an applicator detachably coupled to the upper surface of the mold,
wherein the applicator is formed in a plate shape, and has a first surface which protects the microneedles so that the microneedles are not to be exposed during packaging and a second surface on which adhesion portions are formed to be attached to the microneedles during use and configured so that the microneedles are separated from the mold.

2. The microneedle assembly of Claim 1, wherein the applicator further includes one or more pressing protrusions provided at positions on the second surface corresponding to the microneedles, and having one end portions formed to be flat, and
wherein the adhesion portions are formed on the one end portions of the one or more pressing protrusions.

3. The microneedle assembly of Claim 2, wherein the mold further includes one or more guide protrusions formed outside an area where the microneedles are formed,
wherein the applicator is formed outside an area where the one or more pressing protrusions are formed, and further includes one or more guide holes into which the one or more guide protrusions are inserted,
wherein the mold and the applicator are coupled to each other by the one or more guide protrusions and the one or more guide holes during packaging, and
wherein the one or more guide protrusions and the one or more guide holes are configured to guide the adhesion portions toward the microneedles during use.

4. The microneedle assembly of Claim 1, further comprising: a protection cover configured to be detachably coupled to the second surface of the applicator and temporarily close the second surface during packaging.

5. The microneedle assembly of Claim 1, wherein the applicator is made of a sturdy material.

6. The microneedle assembly of Claim 1, wherein the concave portions of the mold are sharp-tipped concave portions.

7. The microneedle assembly of Claim 1, wherein the microneedles are provided independently of each other.

8. The microneedle assembly of Claim 1, wherein the mold is a primary packaging container in fully close contact with the microneedles and is configured to be removed before the microneedles are applied to a human body.

9. The microneedle assembly of Claim 3, wherein the guide holes are formed to penetrate from the first surface to the second surface of the applicator.
